# EUROPEAN PATENT APPLICATION

(11) **EP 2 898 875 A1**
(43) Date of publication of application: **29.07.2015**
(21) Application number: 15154814.6
(22) Date of filing: 16.09.2011
(51) Int. Cl.: A61K 8/34, A61Q 5/00, C07C 69/612, C07C 235/34

(54) **Novel ortho-diphenol derivatives, cosmetic composition comprising same and cosmetic treatment process**

(30) Priority: 22.09.2010 FR 1057588; 22.09.2010 FR 1057589; 28.09.2010 US 387016 P; 28.09.2010 US 387018 P
(62) Divisional of application: 11757330.3
(71) Applicant: L'OREAL, 75008 Paris (FR)
(72) Inventor: Ramos-Stanbury, Laure, 92330 SCEAUX (FR); Zard, Samir Zard, 91190 GIF SUR YVETTE (FR)
(74) Representative: Dodin, Catherine

(57) **Abstract**

The present invention relates to a cosmetic treatment process for keratin materials, in which a cosmetic composition comprising at least one compound of formula (I), or salts or solvates thereof, is applied to said keratin material : 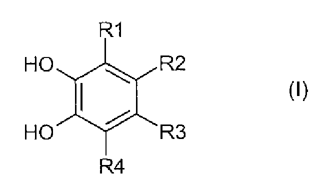 in which R1 to R4, which may be identical or different, denote:
- a hydrogen atom,
- a silicone radical L-(Si(T)₂-O)ₙ-Si(T)₂-CₘH₂ₘ₊₁, or
- a radical -L-(O-(CH₂)ₓ)ₚ-O-CH₃
at least one of the radicals R1, R2, R3 and/or R4 is other than hydrogen.

It is in particular a hair treatment process, in particular for protecting and/or repairing the hair, in particular damaged hair.

The invention also relates to the compounds of formula (I) and to the cosmetic compositions comprising same.

## Description

The present invention relates to a cosmetic treatment process, especially for the hair, and in particular for protecting and/or repairing keratin fibres, especially damaged keratin fibres, said process using particular ortho-diphenol derivatives, especially comprising PEG or silicone chains; the invention also relates to novel ortho-diphenol derivatives and also to the cosmetic compositions comprising same.

It is known that certain hair treatments, such as dyeing or permanent-waving, can attack the hair fibre and especially bring about the loss of some of its constituents that are naturally present, in particular fatty acids. The hair products currently proposed do not, however, make it possible to relipidize in a long-lasting manner the fibre thus damaged.

It is known, moreover, that the cosmetic qualities of the hair can be improved by applying compositions based on active agents that make it possible to confer thereon various properties such as sheen, ease of disentangling, body, suppleness, liveliness or softness. In order to obtain good effectiveness, these active agents should of course exhibit, with respect to the keratin fibres, a certain affinity and also good persistence. In other words, these active agents should, as far as possible, remain attached to the hair in a sufficient amount with a view to conferring thereon the desired properties.

With a view to improving the persistence of these active agents on the hair, a composition comprising lipids bearing functional groups, covalently bonded to the surface of the keratin materials, skin or hair, has in particular been proposed, for example by document WO 2008/156327. The functional groups are very preferentially chosen from hydroxysuccinimidyl ester or maleimide groups, other functional groups, such as thiol or disulphide, also being mentioned. The lipids, for their part, are C8-C28, preferably branched, fatty acids.

One of the objectives of the present invention is to propose novel compounds capable of giving a damaged fibre back the physicochemical surface properties of a natural fibre, in a long-lasting manner. This can thus be referred to as relipidizing the fibre.

It can be considered that the compounds according to the invention will enable the damaged fibre to return to a hydrophobic surface state close to that of the natural hair, in a long-lasting manner. The hair would thus be less sensitive to the changes in physicochemical properties brought about by the penetration of water into the hair fibre. The hair would then be more lively and less brittle. Its general appearance would be improved, the hair would be softer and more shiny.

Moreover, the compounds according to the invention could make it possible to improve the surface condition of keratin fibres, particularly in a humid environment.

The invention relates to novel compounds of formula (I) and to the cosmetic compositions comprising same.

Another subject of the present invention is a cosmetic treatment process for keratin materials, in which a cosmetic composition comprising at least one compound of formula (I), or salts or solvates thereof, as defined hereinafter, is applied to said keratin materials.

In particular, it is a hair treatment process, in particular for damaged hair; preferably, it is a process for protecting and/or repairing the hair, in particular damaged hair.

By virtue of the compounds according to the invention, it would be possible to improve the cosmetic appearance of hair that was damaged, for example following bleaching, permanent-waving, straightening, smoothing or dyeing, or else naturally brittle hair, or alternatively hair attacked, for example, by UV radiation, pollution or repeated brushing. It would also be possible to give the hair body and/or liveliness and to make the surface thereof shiny; it would also be possible to protect and/or repair damaged hair.

The compounds according to the invention therefore correspond to formula (I), and also salts and solvates thereof: 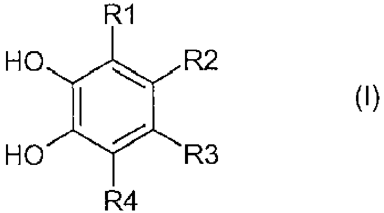 in which R1 to R4, which may be identical or different, denote:
- a hydrogen atom,
- a silicone polymer radical of general formula -L-(Si(T)₂-O)ₙ-Si(T)₂-CₘH₂ₘ₊₁ in which T, which may be identical or different, is a C1-C20 alkyl group, preferably methyl or ethyl; or a C1-C20 alkylamino group, in particular aminoethyl; or an iminopropyl group - (CH₂)₃-NH-(CH₂)₂-NH₂; or an aryl group, in particular phenyl; and preferably a radical
- L- (Si (CH₃)₂-O)ₙ-Si (CH₃)₂-CₘH₂ₘ₊₁ ;
   in which L, representing a linker between the catechol unit and the silicone chain, represents a saturated, linear C1-C40 or branched C3-C40, or unsaturated (having one or more C=C unsaturations) C2-C40 hydrocarbon-based chain; said chain being optionally interrupted with one or more groups chosen from: -O-, -C(O)-, -NH- and NR₇, and combinations thereof, in particular -C(O)O-, -OC(O)-, -C(O)NH-, -NHC(O)-, -NHC(O)NH-and -OC(O)O-; and/or said chain being optionally substituted with one or more groups chosen from -OR₅. -C(O)OR₅, -C(O)NHR₅ and -NR₆R₅, with R₅ and R₆, which may be identical or different, representing a hydrogen atom or a linear or branched C1-C6 alkyl radical; and n = 2 to 3500, in particular n = 2-150; and m = 1 to 6;
- a radical -L-(O-(CH₂)ₓ)ₚ-O-CH₃
   in which L represents a saturated, linear C1-C40 or branched C3-C40, or unsaturated (having one or more C=C unsaturations) C2-C40 hydrocarbon-based chain; said chain being optionally interrupted with one or more groups chosen from: -O-, -C(O)-, -NH- and NR₇, and combinations thereof, in particular -C(O)O-, -OC(O)-, -C(O)NH-, -NHC(O)-, -NHC(O)NH- and -OC(O)O-; and/or said chain being optionally substituted with one or more groups chosen from: -OR₅-, -C(O)OR₅, -C(O)NHR₅ and-NR₆R₅, with R₅ and R₆, which may be identical or different, representing a hydrogen atom or a linear or branched C1-C6 alkyl radical; and x = 2 or 3 and p = 5 to 200;
- in all of these radicals, R7 denoting a hydrogen atom or a C1-C6 alkyl radical, optionally substituted with one or more C1-C4 alkyls and/or OH;
it being understood that at least one of the radicals R1, R2, R3 and/or R4 is other than hydrogen.

Preferably, in formula (I), R1 = R2 = R3 = H or else R1 = R3 = R4 = H.

Preferably, in formula (I), the radical (s) R1 to R4 other than hydrogen are chosen from:
- a radical -L-(Si(CH₃)₂-O)ₙ-Si(CH₃)₂-CₘH₂ₘ₊₁
   in which L represents a saturated linear C1-C30, in particular C6-C30, or even C8-C28, or unsaturated C2-C30, in particular C6-C30, or even C8-C28, hydrocarbon-based chain; optionally interrupted with one or more groups chosen from: - O-, -C(O)-, -NH-, -C(O)O-, -C(O)NH- and -NHC(O)-; and/or optionally substituted with one or more -OH; and n = 2 to 3500, in particular 2 to 150, and m = 1 to 6; or
- a radical -L-(O-(CH₂)ₓ)ₚ-O-CH₃
   in which L represents a saturated linear C1-C30, in particular C6-C30, or even C8-C28, or unsaturated C2-C30, in particular C6-C30, or even C8-C28, hydrocarbon-based chain; optionally interrupted with one or more groups chosen from: - O-, -C(O)-, -NH-, -C(O)O-, -C(O)NH- and -NHC(O)-; and/or optionally substituted with one or more -OH; and x = 2 or 3 and p = 5 to 200.

Preferentially, the compounds of the invention correspond to the following formula (II) or (III), and also salts and/or solvates thereof: 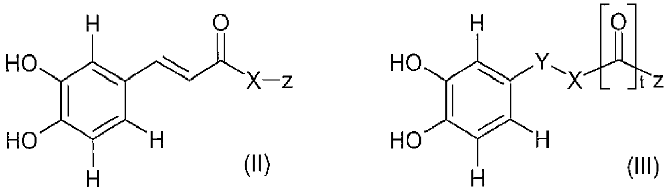 in which:
- X denotes -NR₈- or -O-, with R₈ = H or C1-C4 alkyl;
- Z denotes:
   (i) a radical -L'-(Si(CH₃)₂-O)ₙ-Si(CH₃)₂-CₘH₂ₘ₊₁, in which L' represents a saturated, linear C1-C30 or branched C3-C30, or unsaturated (having one or more unsaturations) C2-C30 hydrocarbon-based chain; said chain being optionally interrupted with one or more groups chosen from: -O-, -C(O)-and -NH-, and combinations thereof, in particular -C(O)O-, - OC(O)-, -C(O)NH- and -NHC(O)-; said chain being optionally substituted with one or more OH; n = 2 to 3500 and m = 1 to 6; or
   (ii) a radical -L'-(O-(CH₂)ₓ)ₚ-O-CH₃, in which L' represents a saturated, linear C1-C30 or branched C3-C30, or unsaturated (having one or more unsaturations) C2-C30 hydrocarbon-based chain; said chain being optionally interrupted with one or more groups chosen from: -O-, -C(O)- and -NH-, and combinations thereof, in particular -C(O)O-, -OC(O)-, -C(O)NH- and -NHC(O)-; said chain being optionally substituted with one or more OH; x = 2 or 3 and p = 5 to 200;
- t = 0 or 1;
- Y denotes a saturated linear C1-C10 or branched C2-C10 hydrocarbon-based chain.

Preferably, in formula (II):
- X = O or NH; and/or
- Z denotes:
   (i) a radical -L'-(Si(CH₃)₂-O)ₙ-Si(CH₃)₂-CₘH₂ₘ₊₁, in which L' represents a saturated, linear C1-C12 or branched C3-C12, or unsaturated (having one or more unsaturations) C2-C12 hydrocarbon-based chain; said chain being optionally interrupted with one or more groups chosen from: -O-, -NH-, - C(O)O-, -OC(O)-, -C(O)NH- and -NHC(O)-; said chain being optionally substituted with one or more OH; n = 2 to 3500, in particular 2 to 150, and m = 1 to 6, in particular 2 to 5; or
   (ii) a radical -L'-(O-(CH₂)ₓ)ₚ-O-CH₃, in which L' represents a saturated, linear C1-C12 or branched C3-C12, or unsaturated (having one or more unsaturations) C2-C12 hydrocarbon-based chain, preferably saturated linear C1-C6 hydrocarbon-based chain; x = 2 and p = 5 to 200.

Preferably, in formula (III):
- X = O or NH; and/or
- Y denotes a saturated linear C1-C4 hydrocarbon-based chain, preferentially -CH₂-CH₂-; and/or
- Z denotes:
   (i) a radical -L'-(Si(CH₃)₂-O)ₙ-Si(CH₃)₂-CₘH₂ₘ₊₁, in which L' represents a saturated, linear C1-C12 or branched C3-C12, or unsaturated (having one or more unsaturations) C2-C12 hydrocarbon-based chain; said chain being optionally interrupted with one or more groups chosen from: -O-, -NH-, - C(O)O-, -OC(O)-, -C(O)NH- and -NHC(O)-; said chain being optionally substituted with one or more OH; n = 2 to 3500, in particular 2 to 150, and m = 1 to 6, in particular 2 to 5; or
   (ii) a radical -L'-(O-(CH₂)ₓ)ₚ-O-CH₃, in which L' represents a saturated, linear C1-C12 or branched C3-C12, or unsaturated (having one or more unsaturations) C2-C12 hydrocarbon-based chain, preferably saturated linear C1-C6 hydrocarbon-based chain; x = 2 and p = 5 to 200.

Among the compounds corresponding to formula (I), mention may be made of the following compounds, and also salts and/or solvates thereof:

| | |
|---|---|
| 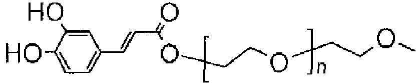 | 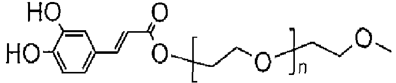 |
| n=10 (PEG 500) | PEG5000 |
| | |
| 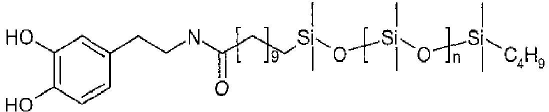 | |
| MW = 1600 | |
| 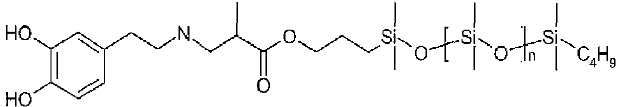 | |
| MW = 750, 1150, 5000, 10 000 | |
| 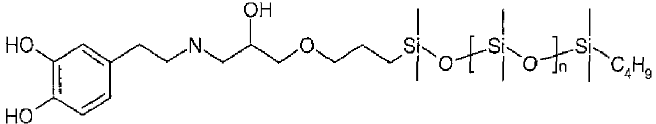 | |
| MW = 1150, 5000 | |
| 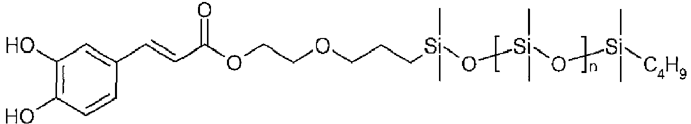 | |
| MW = 1150, 5000, 10 000 | |
| 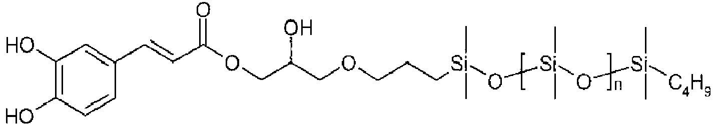 | |
| MW = 1150, 5000 | |
| 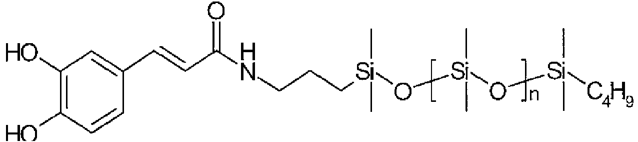 | |
| MW = 460-510 | |

A mixture of compounds of formula (I) can quite obviously be used.

The salts and solvates of the compounds of formula (I) also form part of the present invention.

The salts comprise the conventional nontoxic salts of said compounds, such as those formed from organic or inorganic acids. Mention may be made of the salts of inorganic acids, such as sulphuric acid, hydrochloric acid, hydrobromic acid, hydriodic acid, phosphoric acid or boric acid. Mention may also be made of the salts of organic acids, which can comprise one or more carboxylic, sulphonic or phosphonic acid groups. They may be linear, branched or cyclic aliphatic acids or else aromatic acids. These acids may also comprise one or more heteroatoms chosen from O and N, for example in the form of hydroxyl groups. Mention may in particular be made of propionic acid, acetic acid, terephthalic acid, citric acid and tartaric acid. Among the salts of organic or inorganic bases, mention may be made of the triethanolamine, amidopropanediol, sodium, zinc or calcium salts. The cosmetically acceptable solvates of the compounds according to the invention comprise conventional solvates such as those formed during the final step of preparation of said compounds owing to the presence of solvents. Mention may be made of the solvates owing to the presence of water or of linear of branched alcohols such as ethanol or isopropanol.

The compounds of formula (I) can be obtained according to the synthesis routes described below.

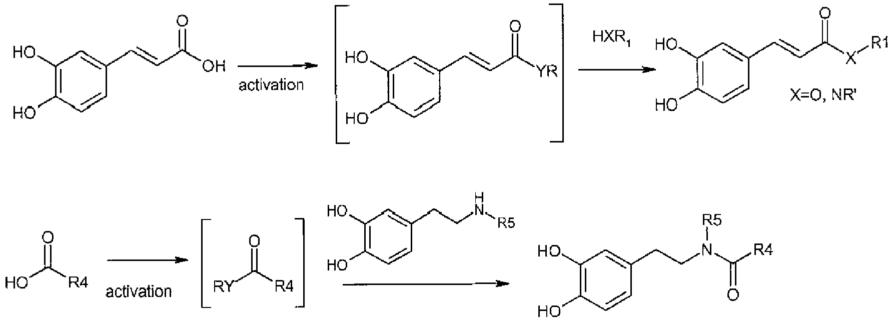

The functionalization of the COOH function to give a COYR function can be carried out according to the conventional methods for activating acids, in particular described in "Comprehensive Organic Transformation by R. Larock, Wiley 5th Ed.", in the chapter "Interconversion of nitriles, carboxylic acids and derivatives"; the reaction of the activated form with RYH is then carried out. Preferably, the activation methods are the formation of methyl ester or of acid chloride, for example by using thionyl chloride or oxalyl chloride, or 1-chloro-N,N-2-trimethyl-1-propenamine; or the formation of mixed anhydride, for example using alkyl chloroformates; or else the use of carbodiimides, such as, for example, DCC, DIC or EDC; or else of diethylcyanophosphate as described in "Phosphorus in organic synthesis-XI, Amino acids and peptides-XXI, Reaction of diethyl phosphorocyanidate with carboxylic acids. A new synthesis of carboxylic esters and amides, Tetrahedron, 32, 1976, 2211-2217".

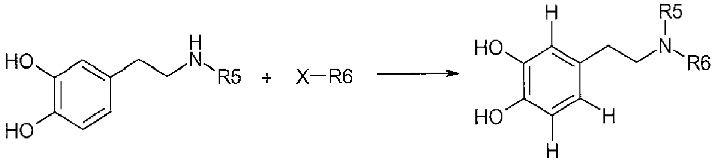

An alkylating agent such as, for example, a haloalkane, an alkyl mesylate or an alkyl tosylate is reacted with the amine. An ion exchange can be carried out if necessary at the end of the reaction, by contact with an ion exchange resin chosen according to the exchanges desired. These resins are, for example, IRA 402 (alkyl sulphates-to-chlorides exchange), and IRA 400 (iodide-to-chloride exchange). The alkylation can also be carried out by reductive alkylation as described in Advanced Organic Chemistry, J March, 4th Ed., p. 898-900; or else by addition on a double bond, for example of Mickaël type as described in Advanced Organic Chemistry, J March, 4th Ed., p. 768-770.

The compounds of formula (I) find quite particular application in the cosmetics, in particular hair, field. They may be present in the composition in solubilized form, for example in water or an organic solvent, in particular ethanol, benzyl alcohol, butyl acetate, ethyl acetate, isopropyl myristate, PEGs (polyethylene glycols) that are liquid at 25°C, isododecane, plant oils, liquid petroleum jelly, or else in a silicone solvent, such as volatile silicone oils, in particular D5; and also mixtures thereof. They may also be present in the form of an aqueous or organic dispersion or a dispersion in silicone material, in particular in one of these solvents.

They can be used in the cosmetic compositions, in particular hair compositions, in a proportion of from 0.001 to 20% by weight of dry matter, in particular from 0.1 to 15% by weight, or even from 1 to 10% by weight, and even better still from 2 to 5% by weight, relative to the total weight of the composition.

The cosmetic compositions according to the invention comprise a cosmetically acceptable medium, i.e. a medium that is compatible with keratin materials such as facial or body skin, the lips, the hair, the eyelashes, the eyebrows and the nails.

The composition can thus comprise a hydrophilic medium comprising water and/or one or more hydrophilic organic solvents, such as alcohols and in particular linear or branched C1-C6 monoalcohols, such as ethanol, tert-butanol, n-butanol, isopropanol or n-propanol, and polyols such as glycerol, diglycerol, propylene glycol, sorbitol, pentylene glycol, hexylene glycol, and polyethylene glycols, or alternatively ethers of glycols, in particular C₂ glycols, and C₂-C₄ aldehydes which are hydrophilic.

The composition can also comprise a fatty phase, in particular consisting of fatty substances that are liquid at ambient temperature (25°C in general) and/or of fatty substances that are solid at ambient temperature, such as waxes, pasty fatty substances, gums and mixtures thereof. These fatty substances may be of animal, plant, mineral or synthetic origin. This fatty phase may also contain lipophilic organic solvents.

As fatty substances that are liquid at ambient temperature, often referred to as oils, that can be used in the invention, mention may be made of: hydrocarbon-based oils of animal origin, such as perhydrosqualene; hydrocarbon-based plant oils, such as liquid triglycerides of fatty acids containing 4 to 10 carbon atoms, for instance heptanoic or octanoic acid triglycerides, or else sunflower oil, maize oil, soya oil, grapeseed oil, sesame oil, apricot oil, macadamia oil, castor oil, avocado oil, caprylic/capric acid triglycerides, jojoba oil, shea butter; linear or branched hydrocarbons of mineral or synthetic origin, such as liquid paraffins and derivatives thereof, petroleum jelly, polydecenes, hydrogenated polyisobutene such as parleam; synthetic esters and ethers, in particular of fatty acids, for instance purcellin oil, isopropyl myristate, 2-ethylhexyl palmitate, 2-octyldodecyl stearate, 2-octyldodecyl erucate, isostearyl isostearate; hydroxylated esters, for instance isostearyl lactate, octyl hydroxystearate, octyldodecyl hydroxystearate, diisostearyl malate, triisocetyl citrate, fatty alcohol heptanoates, octanoates and decanoates; polyol esters, for instance propylene glycol dioctanoate, neopentyl glycol diheptanoate, diethylene glycol diisononanoate; pentaerythritol esters; fatty alcohols having 12 to 26 carbon atoms, for instance octyldodecanol, 2-butyloctanol, 2-hexyldecanol, 2-undecyl pentadecanol, oleyl alcohol; partially hydrocarbon-based and/or silicone-based fluoro oils; silicone oils, for instance volatile or non-volatile, linear or cyclic polydimethylsiloxanes (PDMSs) which are liquid or pasty at ambient temperature (25°C), such as cyclomethicones, dimethicones, optionally comprising a phenyl group, for instance phenyl trimethicones, phenyl trimethylsiloxydiphenylsiloxanes, diphenylmethyldimethyltrisiloxanes, diphenyl dimethicones, phenyl dimethicones, polymethylphenylsiloxanes; mixtures thereof.

The composition according to the invention can also comprise one or more physiologically acceptable organic solvents. Mention may in particular be made, in addition to the hydrophilic organic solvents mentioned above, of ketones that are liquid at ambient temperature, such as methyl ethyl ketone, methyl isobutyl ketone, diisobutyl ketone, isophorone, cyclohexanone or acetone; propylene glycol ethers that are liquid at ambient temperature, such as propylene glycol monomethyl ether, propylene glycol monomethyl ether acetate, dipropylene glycol mono-n-butyl ether; short-chain esters (containing 3 to 8 carbon atoms in total), such as ethyl acetate, methyl acetate, propyl acetate, n-butyl acetate or isopentyl acetate; ethers that are liquid at 25°C, such as diethyl ether, dimethyl ether or dichlorodiethyl ether; alkanes that are liquid at 25°C, such as decane, heptane, dodecane, isododecane or cyclohexane; aldehydes that are liquid at 25°C, such as benzaldehyde and acetaldehyde, and mixtures thereof.

The composition according to the invention can also comprise at least one ingredient commonly used in the cosmetics industry, such as vitamins, fragrances, pearlescent agents, thickeners, gelling agents, trace elements, emollients, sequestering agents, basifying or acidifying agents, preservatives, sunscreens, surfactants, emulsifiers, antioxidants, anti-hair loss agents, antidandruff agents, propellants, ceramides, polymers, in particular film-forming polymers; fillers, nacres, colorants and in particular pigments and dyes; reducing agents, and also mixtures thereof. Of course, those skilled in the art will take care to select this or these optional additional compound(s), and/or the amount thereof, in such a way that the advantageous properties of the composition according to the invention are not, or not substantially, impaired by the addition envisaged.

Those skilled in the art will choose the appropriate galenical form, and also the method for preparing same, on the basis of their general knowledge, taking into account, firstly, the nature of the constituents used, in particular their solubility in the support, and secondly, the application envisaged for the composition. Thus, the composition according to the invention may be in the form of a suspension, a dispersion, in particular of oil in water by virtue of vesicles; an aqueous, aqueous-alcoholic or oily solution optionally thickened or even gelled; an oil-in-water, water-in-oil or multiple emulsion; an aqueous or aqueous-alcoholic gel, a foam; an oily or emulsified gel; a dispersion of vesicles, in particular lipid vesicles; a two-phase or multiphase lotion; a spray. This composition can have the appearance of a lotion, a cream, an ointment, a soft paste, a salve, a solid cast or moulded especially as a stick or in a dish, or else a compacted solid.

The cosmetic composition according to the invention may be in the form of a product for caring for and/or making up bodily or facial skin, the lips, the nails, the eyelashes and/or the hair, an antisun or self-tanning product, or a hair product.

It especially has a particularly advantageous application in the hair care field, in particular for protecting and/or repairing keratin fibres, in particular damaged keratin fibres (hair).

The hair compositions are preferably shampoos, gels, hair setting lotions, blow-drying lotions, hair styling and fixing compositions such as lacquers or sprays, hair care or hair repair compositions, such as rinse-out or leave-in conditioners, rinse-out or leave-in hair care products, aqueous or aqueous-alcoholic lotions for skin and/or hair care; sprays, in particular leave-in sprays; permanent-wave, hair straightening, dyeing or bleaching compositions, or else in the form of rinse-out compositions to be applied before or after dyeing, bleaching, permanent-waving or relaxing the hair, or alternatively between the two steps of a permanent-waving or smoothing operation.

The compositions may also be in the form of shower gels or bubble baths.

In one preferred embodiment, the compositions in accordance with the invention can be used for washing, caring for, treating, protecting and/or repairing keratin materials such as the hair, the skin, the eyelashes, the eyebrows, the nails, the lips or the scalp, and more particularly the hair, especially damaged hair.

A subject of the invention is also a cosmetic treatment process for keratin materials such as the skin, the lips, the eyelashes, the eyebrows, the nails or the hair, especially a hair treatment process, and in particular a process for protecting and/or repairing the hair, in which a cosmetic composition comprising at least one compound of formula (I) is applied to said keratin materials.

Most particularly, it is a process for protecting and/or repairing the hair, in particular damaged hair.

It is possible to envisage a post-treatment of the hair after the application of the composition according to the invention, for example the application of heat and/or of UV radiation and/or of an oxidizing agent such as aqueous hydrogen peroxide.

## Claims

1. Compound having the following formula (I), and salts and solvates thereof: 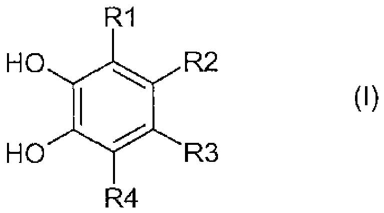 in which R1 to R4, which may be identical or different, denote:
- a hydrogen atom,
- a silicone polymer radical of general formula -L-(Si(T)₂-O)ₙ-Si(T)₂-CₘH₂ₘ₊₁ in which T, which may be identical or different, is a C1-C20 alkyl group, preferably methyl or ethyl; or a C1-C20 alkylamino group, in particular aminoethyl; or an iminopropyl group - (CH₂)₃-NH-(CH₂)₂-NH₂; or an aryl group, in particular phenyl; and preferably a radical -L- (Si (CH₃)₂-O)ₙ-Si (CH₃)₂-CₘH₂ₘ₊₁ ;
in which L, representing a linker between the catechol unit and the silicone chain, represents a saturated, linear C1-C40 or branched C3-C40, or unsaturated (having one or more C=C unsaturations) C2-C40 hydrocarbon-based chain; said chain being optionally interrupted with one or more groups chosen from: -O-, -C(O)-, -NH- and NR₇. and combinations thereof, in particular -C(O)O-, -OC(O)-, -C(O)NH-, -NHC(O)-, -NHC(O)NH-and -OC(O)O-; and/or said chain being optionally substituted with one or more groups chosen from -OR₅. -C(O)OR₅, -C(O)NHR₅ and -NRr₆R₅, with R₅ and R₆, which may be identical or different, representing a hydrogen atom or a linear or branched C1-C6 alkyl radical; and n = 2 to 3500, in particular n = 2-150; and m = 1 to 6;
- a radical -L-(O-(CH₂)ₓ)ₚ-O-CH₃
in which L represents a saturated, linear C1-C40 or branched C3-C40, or unsaturated (having one or more C=C unsaturations) C2-C40 hydrocarbon-based chain; said chain being optionally interrupted with one or more groups chosen from: -O-, -C(O)-, -NH- and NR₇, and combinations thereof, in particular -C(O)O-, -OC(O)-, -C(O)NH-, -NHC(O)-, -NHC(O)NH- and -OC(O)O-;
and/or said chain being optionally substituted with one or more groups chosen from: -OR₅-, -C(O)OR₅, -C(O)NHR₅ and-NR₆R₅, with R₅ and R₆, which may be identical or different, representing a hydrogen atom or a linear or branched C1-C6 alkyl radical; and x = 2 or 3 and p = 5 to 200;
- R7 denoting a hydrogen atom or a C1-C6 alkyl radical, optionally substituted with one or more C1-C4 alkyls and/or OH;
it being understood that at least one of the radicals R1, R2, R3 and/or R4 is other than hydrogen.

2. Compound according to Claim 1, in which, in formula (I), R1 = R2 = R3 = H or R1 = R3 = R4 = H.

3. Compound according to one of the preceding claims, in which, in formula (I), the radical (s) R1 to R4 other than hydrogen is/are chosen from:
- a radical -L-(Si(CH₃)₂-O)ₙ-Si(CH₃)₂-CₘH₂ₘ₊₁
in which L represents a saturated linear C1-C30, in particular C6-C30, or even C8-C28, or unsaturated C2-C30, in particular C6-C30, or even C8-C28, hydrocarbon-based chain; optionally interrupted with one or more groups chosen from: - O-, -C(O)-, -NH-, -C(O)O-, -C(O)NH- and -NHC(O)-; and/or optionally substituted with one or more -OH; and n = 2 to 3500, in particular 2 to 150, and m = 1 to 6; or
- a radical -L-(O-(CH₂)ₓ)ₚ-O-CH₃
in which L represents a saturated linear C1-C30, in particular C6-C30, or even C8-C28, or unsaturated C2-C30, in particular C6-C30, or even C8-C28, hydrocarbon-based chain; optionally interrupted with one or more groups chosen from: - O-, -C(O)-, -NH-, -C(O)O-, -C(O)NH- and -NHC(O)-; and/or optionally substituted with one or more -OH; and x = 2 or 3 and p = 5 to 200.

4. Compound according to one of the preceding claims, corresponding to the following formula (II) or (III), and also salts and/or solvates thereof: 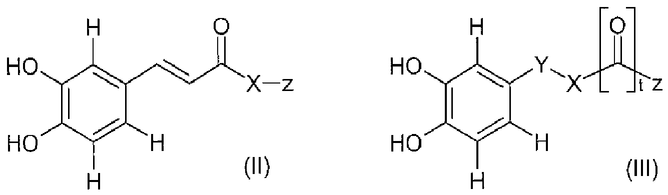 in which:
- X denotes -NR₈- or -O-, with R₈ = H or C1-C4 alkyl;
- Z denotes:
(i) a radical -L'-(Si(CH₃)₂-O)ₙ-Si(CH₃)₂-CₘH₂ₘ₊₁, in which L' represents a saturated, linear C1-C30 or branched C3-C30, or unsaturated (having one or more unsaturations) C2-C30 hydrocarbon-based chain; said chain being optionally interrupted with one or more groups chosen from: -O-, -C(O)-and -NH-, and combinations thereof, in particular -C(O)O-, - OC(O)-, -C(O)NH- and -NHC(O)-; said chain being optionally substituted with one or more OH; n = 2 to 3500 and m = 1 to 6; or
(ii) a radical -L'-(O-(CH₂)ₓ)ₚ-O-CH₃, in which L' represents a saturated, linear C1-C30 or branched C3-C30, or unsaturated (having one or more unsaturations) C2-C30 hydrocarbon-based chain; said chain being optionally interrupted with one or more groups chosen from: -O-, -C(O)- and -NH-, and combinations thereof, in particular -C(O)O-, -OC(O)-, -C(O)NH- and -NHC(O)-; said chain being optionally substituted with one or more OH; x = 2 or 3 and p = 5 to 200;
- t = 0 or 1;
- Y denotes a saturated linear C1-C10 or branched C2-C10 hydrocarbon-based chain.

5. Compound according to claim 4, in which, in formula (II):
- X = O or NH; and/or
- Z denotes:
(i) a radical -L'-(Si(CH₃)₂-O)ₙ-Si(CH₃)₂-CₘH₂ₘ₊₁, in which L' represents a saturated, linear C1-C12 or branched C3-C12, or unsaturated (having one or more unsaturations) C2-C12 hydrocarbon-based chain; said chain being optionally interrupted with one or more groups chosen from: -O-, -NH-, - C(O)O-, -OC(O)-, -C(O)NH- and -NHC(O)-; said chain being optionally substituted with one or more OH; n = 2 to 3500, in particular 2 to 150, and m = 1 to 6, in particular 2 to 5; or
(ii) a radical -L'-(O-(CH₂)ₓ)ₚ-O-CH₃, in which L' represents a saturated, linear C1-C12 or branched C3-C12, or unsaturated (having one or more unsaturations) C2-C12 hydrocarbon-based chain, preferably saturated linear C1-C6 hydrocarbon-based chain; x = 2 and p = 5 to 200.

6. Compound according to claim 4, in which, in formula (III):
- X = O or NH; and/or
- Y denotes a saturated linear C1-C4 hydrocarbon-based chain, preferentially -CH₂-CH₂-; and/or
- Z denotes:
(i) a radical -L'-(Si(CH₃)₂-O)ₙ-Si(CH₃)₂-CₘH₂ₘ₊₁, in which L' represents a saturated, linear C1-C12 or branched C3-C12, or unsaturated (having one or more unsaturations) C2-C12 hydrocarbon-based chain; said chain being optionally interrupted with one or more groups chosen from: -O-, -NH-, - C(O)O-, -OC(O)-, -C(O)NH- and -NHC(O)-; said chain being optionally substituted with one or more OH; n = 2 to 3500, in particular 2 to 150, and m = 1 to 6, in particular 2 to 5; or
(ii) a radical -L'-(O-(CH₂)ₓ)ₚ-O-CH₃, in which L' represents a saturated, linear C1-C12 or branched C3-C12, or unsaturated (having one or more unsaturations) C2-C12 hydrocarbon-based chain, preferably saturated linear C1-C6 hydrocarbon-based chain; x = 2 and p = 5 to 200.

7. Compound according to one of the preceding claims, chosen from the following compounds, and also salts and/or solvates thereof:

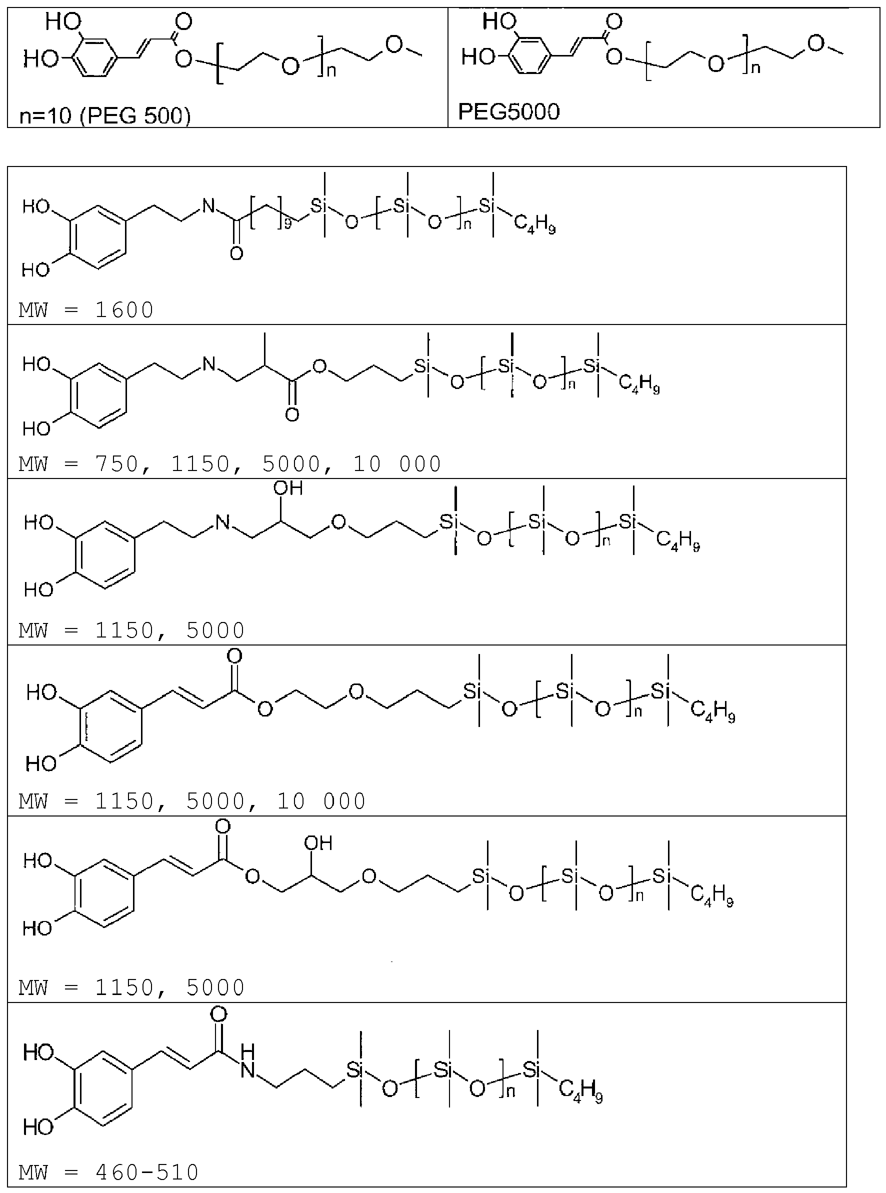

8. Cosmetic composition comprising, in a cosmetically acceptable medium, at least one compound of formula (I) as defined in one of the preceding claims.

9. Composition according to Claim 8, in which the compounds of formula (I) are present in solubilized form, for example in water or an organic solvent, in particular ethanol, benzyl alcohol, butyl acetate, ethyl acetate, isopropyl myristate, PEGs (polyethylene glycols) that are liquid at 25°C, isododecane, plant oils, liquid petroleum jelly, or else in a silicone solvent, such as volatile silicone oils, in particular D5; and also mixtures thereof; or in the form of an aqueous or organic dispersion or a dispersion in a silicone substance, in particular in one of these solvents.

10. Composition according to one of Claims 8 or 9, in which the compounds of formula (I) are present in the composition in a proportion of from 0.001 to 20% by weight of dry matter, in particular from 0.1 to 15% by weight, or even from 1 to 10% by weight, and better still from 2 to 5% by weight, relative to the total weight of the composition.

11. Composition according to one of Claims 8 to 10, in which the cosmetically acceptable medium comprises at least one ingredient chosen from water, hydrophilic organic solvents, waxes, pasty fatty substances, gums, oils, lipophilic organic solvents, vitamins, fragrances, pearlescent agents, thickeners, gelling agents, trace elements, emollients, sequestering agents, basifying or acidifying agents, preservatives, sunscreens, surfactants, emulsifiers, antioxidants, anti-hair loss agents, antidandruff agents, propellants, ceramides, polymers, in particular film-forming polymers; fillers, nacres, colorants, and in particular pigments and dyes; reducing agents, and also mixtures thereof.

12. Composition according to one of Claims 8 to 11, which is in the form of a product for caring for and/or making up bodily or facial skin, the lips, the nails, the eyelashes and/or the hair, an antisun or self-tanning product, or a hair product, in particular for protecting and/or repairing keratin fibres, in particular damaged keratin fibres (hair).

13. Cosmetic treatment process for keratin materials, in which a cosmetic composition according to one of claims 8 to 12 is applied to said keratin materials.

14. Process according to claim 13, consisting of a hair treatment process, most particularly for damaged hair; in particular a process for protecting and/or repairing the hair, most particularly damaged hair.
